(19) Europäisches Patentamt / European Patent Office / Office européen des brevets

(11) **EP 3 194 952 B1**

(12) **FASCICULE DE BREVET EUROPEEN**

(45) Date de publication et mention de la délivrance du brevet:
**22.05.2024 Bulletin 2024/21**

(21) Numéro de dépôt: **15742333.6**

(22) Date de dépôt: **03.08.2015**

(51) Classification Internationale des Brevets (IPC):
**G01N 27/447** *(2006.01)* **G01N 30/00** *(2006.01)*
**B01L 3/00** *(2006.01)*

(52) Classification Coopérative des Brevets (CPC):
**G01N 30/0005; B01L 3/502; B01L 3/502753;**
**G01N 27/44791;** B01L 2300/0816;
B01L 2400/0421; G01N 2030/004

(86) Numéro de dépôt international:
**PCT/EP2015/067826**

(87) Numéro de publication internationale:
**WO 2016/016470 (04.02.2016 Gazette 2016/05)**

(54) **PROCÉDÉ ET DISPOSITIF DE CONCENTRATION DE MOLÉCULES OU OBJETS DISSOUS EN SOLUTION**

VERFAHREN UND VORRICHTUNG ZUR KONZENTRATION VON IN EINER LÖSUNG AUFGELÖSTEN MOLEKÜLEN ODER OBJEKTEN

METHOD AND DEVICE FOR CONCENTRATING MOLECULES OR OBJECTS DISSOLVED IN SOLUTION

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **01.08.2014 FR 1457544**

(43) Date de publication de la demande:
**26.07.2017 Bulletin 2017/30**

(73) Titulaire: **Centre National de la Recherche Scientifique**
**75794 Paris Cedex 16 (FR)**

(72) Inventeurs:
• **BANCAUD, Aurélien**
**31400 Toulouse (FR)**
• **RANCHON, Hubert**
**31400 Toulouse (FR)**
• **LEICHLE, Thierry**
**31400 Toulouse (FR)**
• **TEERAPANICH, Pattamon**
**31400 Toulouse (FR)**

(74) Mandataire: **Atout PI Laplace**
**Immeuble Up On**
**25 Boulevard Romain Rolland**
**CS 40072**
**75685 Paris Cedex 14 (FR)**

(56) Documents cités:
WO-A1-2014/020271    US-A1- 2005 126 914
US-A1- 2008 087 546

## Description

## Domaine technique de l'invention

L'invention concerne un procédé de concentration de molécules et d'objets électriquement chargés en solution, ainsi qu'un dispositif apte à la mise en oeuvre de ce procédé. Elle s'inscrit dans le cadre d'une évolution d'une technique de séparation de molécules déformables en solution décrite dans WO2014/020271.

## Art antérieur

On connaît dans l'art antérieur différents procédés et dispositifs permettant de concentrer des molécules, notamment des molécules d'ADN.
Parmi ces procédés et dispositifs, certains, notamment ceux décrits dans US8753492, US8440063, US8783466, mettent en oeuvre des nanocanaux à sélectivité ionique. Ces techniques présentent l'inconvénient de ne pouvoir être mises en oeuvre qu'avec des débits d'écoulement très faibles et sous certaines conditions de salinité.
US 2008/087546 A1 divulgue un dispositif pour la séparation des objets chargés. Le canal d'écoulement comprend les électrodes, qui sont séparés du canal d'écoulement par une membrane.
US 2005/126914 A1 divulgue un séparateur en deux parties.

## Objectifs de l'invention

L'invention a pour objectif de proposer une alternative aux procédés et dispositifs de l'art antérieur.
Notamment un objectif de la présente invention est de proposer une technique permettant de concentrer des molécules ou, d'une manière générale, des objets dissous électriquement chargés avec des débits plus élevés, adaptés à l'analyse d'échantillons à grande échelle, sans restriction quant aux conditions de salinités.
Les objets dissous électriquement chargés peuvent être déformables, et se présenter sous forme de molécules ou d'autres objets plus complexes, tels que des assemblages supramoléculaires ou des cellules ou des fragments de cellules, susceptibles de se déformer sous l'effet d'une contrainte mécanique telle qu'un flux hydrodynamique. De préférence la déformabilité est mesurée par le module d'Young de l'objet, qui doit être inférieur ou égal à $10^9$ Pa, ou selon des modes de réalisation inférieur ou égal à $10^8$ Pa. Le module d'Young peut être mesuré par contact mécanique, par exemple par microscopie à force atomique. A titre d'exemple, le module d'Young de l'ADN vaut typiquement environ 300 MPa, celui de cellules de mammifères de 0,1 à 100 kPa environ. Les objets dissous électriquement chargés peuvent aussi être non déformables et se présenter alors notamment sous forme de nanoparticules ou nanoobjets chargés électriquement.

Dans le cadre de la présente demande, sont notamment considérés comme des objets déformables : les molécules d'ADN simple ou double brin ou d'ARN comprenant au moins 20 bases ou paires de bases (par exemple au moins 1000) ; les peptides, polypeptides ou protéines comprenant au moins 100 motifs d'acides aminés (par exemple au moins 200) ; les glucides polymériques ou autres polymères ; les cellules caryotes ou procaryotes. Il a été démontré que l'invention fonctionne également pour des objets non-déformables tels que des microbilles en matériau expansé tel que le polystyrène.
Un autre objectif de la présente invention est de divulguer une technique qui, dans certains modes de réalisation, permet à la fois de concentrer des molécules ou objets électriquement chargés mais aussi de séparer différentes molécules ou objets électriquement chargés d'un mélange.
Encore un autre objectif de la présente invention est de décrire une technique dont les moyens de mise en oeuvre sont techniquement très simples.

## Exposé de l'invention

Ces objectifs, ainsi que d'autres qui apparaîtront par la suite, sont atteints grâce à l'invention qui concerne un procédé de concentration d'objets électriquement chargés en milieu liquide selon la revendication 1, comprenant :
l'introduction d'un échantillon contenant des objets électriquement chargés dans un canal présentant un axe d'écoulement, une première section transversale orthogonale à l'axe d'écoulement et au moins une deuxième section transversale orthogonale à l'axe d'écoulement, la dimension minimale de ladite seconde section étant inférieure à la dimension correspondante de ladite première section ; l'application d'un flux hydrodynamique dans un sens dudit canal conjointement à l'application dans le sens inverse d'un champ électrique dans ledit canal, permettant de déplacer les objets électriquement chargés dans le canal selon l'axe d'écoulement de la première section vers la deuxième section et de les stopper et les concentrer dans au moins une zone en amont de ladite deuxième section transversale.
Selon l'invention, la deuxième section transversale forme une constriction qui permet de moduler spatialement les champs hydrostatiques et électriques de manière à stopper le déplacement des objets à un endroit prédéterminé et à concentrer les objets à cet endroit. La concentration des molécules ou objets électriquement chargés se fait audit endroit suite à l'accumulation des molécules ou objets électriquement chargés de l'échantillon ayant étant stoppées.
La présente invention permet de surmonter les inconvénients de l'état de la technique. Notamment elle peut être mise en oeuvre avec des débits d'écoulement plus élevés quelles que soient les conditions de salinité. Elle fournit plus particulièrement une méthode de concentration de molécules et autres objets électriquement chargés nécessitant une durée de concentration faible, plus simple et flexible à mettre en oeuvre que les méthodes de l'état de la technique.

On notera que le milieu liquide est non newtonien. On entend dans la présente description par "fluide newtonien", un fluide pour lequel il existe une relation linéaire entre la contrainte mécanique imposée (force exercée sur le fluide par unité de surface) et le cisaillement du fluide (c'est-à-dire gradient de vitesse du fluide). Un " fluide non-newtonien " est donc un fluide qui n'est pas un fluide newtonien.

Par exemple, un fluide non-newtonien selon l'invention peut avoir un coefficient de viscosité dépendant du cisaillement (fluide stationnaire) ; ou il peut avoir un comportement élastique. Selon un mode de réalisation, le fluide est viscoélastique.

Préférentiellement, le milieu liquide présente une viscosité de 1 à 100 cP (centipoise), de préférence de 2 à 20 cP, et de manière encore plus préférée de 2 à 10 cP à température ambiante. Sauf mention contraire, les valeurs de viscosité mentionnées dans la demande sont des valeurs de viscosité statique. Selon un mode de réalisation, le milieu liquide comprend des polymères non chargés, de préférence choisis parmi la polyvinylpyrrolidone (PVP), le poly-(éthylène glycol) le polyacrylamide et leurs mélanges.

Le terme "non chargé " signifie que les polymères en question ont une charge électrostatique globale essentiellement nulle dans le milieu liquide susmentionné. La présence de tels polymères par exemple dans une solution aqueuse permet de rendre le milieu liquide non newtonien (par exemple viscoélastique).

Selon un mode de réalisation, les polymères non chargés sont présents dans une concentration massique de 0,1 à 10 %, de préférence de 0,5 à 5 %, de manière plus particulièrement préférée de 1 à 4 %.

De préférence, la concentration des polymères non-chargés est supérieure ou égale à la concentration critique de recouvrement (concentration à partir de laquelle les polymères sont en contact).

Avantageusement, les polymères non chargés présentent une masse moléculaire moyenne (en masse) de 10 à 100 000 kDa, de préférence de 50 à 10 000 kDa, de manière plus particulièrement préférée de 100 à 1 000 kDa.

Selon une variante préférentielle du procédé selon l'invention, le champ électrique appliqué vaut de 10 V/m à 10000 V/m, de préférence de 100 V/m à 5000 V/m, et de manière plus particulièrement préférée de 200 V/m à 1000 V/m ; et / ou, le flux hydrodynamique est caractérisé par une vitesse moyenne de 1 à 10 000 $\mu$m/s, de préférence de 5 à 5 000 $\mu$m/s et de manière plus particulièrement préférée de 10 à 1 000 $\mu$m /s.

Egalement préférentiellement, l'introduction des objets électriquement chargés est effectuée dans une zone d'introduction du canal et le déplacement des objets électriquement chargés est effectué de la zone d'introduction vers une zone de détection du canal, le procédé comprenant en outre : la détection des objets électriquement chargés arrivant dans la zone de détection.

Avantageusement, les dits objets électriquement chargés sont choisis dans le groupe constitué par : les molécules d'ADN simple ou double brin ou d'ARN comprenant au moins 20 bases ou paires de bases (par exemple au moins 1000) ; les peptides, polypeptides ou protéines comprenant au moins 100 motifs d'acides aminés (par exemple au moins 200) ; les glucides polymériques ou autres polymères ; les cellules caryotes ou procaryotes; les nanoobjets ou nanoparticules.

L'invention concerne également un dispositif de concentration d'objets électriquement chargés en milieu liquide selon la revendication 8, comprenant un canal présentant un axe d'écoulement, le canal étant rempli d'un milieu liquide non newtonien ; des moyens d'application d'un flux hydrodynamique dans le canal ; et des moyens d'application d'un champ électrique dans le canal, où ledit canal présente une première section transversale orthogonale à l'axe d'écoulement et au moins une deuxième section transversale orthogonale à l'axe d'écoulement, la dimension minimale de ladite seconde section étant inférieure à la dimension correspondante de ladite première section.

Selon une variante de l'invention, ledit canal a la forme d'un tube creux de section rectangulaire présentant une paroi inférieure, une paroi supérieure et deux parois latérales, lesdites parois latérales formant localement au moins une constriction.

Préférentiellement, ladite deuxième section présente une dimension minimale inférieure d'au moins 10% ou de 20% ou de 50% ou de 95% à la dimension correspondante de ladite première section.

Avantageusement, lesdites parois latérales dudit tube creux présentent chacune une portion formant un angle compris entre 10° et 65 ° avec ledit axe d'écoulement.

Selon une autre variante ledit canal est la lumière d'un capillaire présentant au moins une constriction.

Dans ce cas, ledit capillaire présente préférentiellement une section circulaire.

Avantageusement, ledit capillaire présente une portion formant un angle compris entre 10° et 65 ° avec ledit axe d'écoulement.

**Liste des figures**

L'invention, ainsi que les différents avantages qu'elle présente seront plus facilement compris grâce à la description qui va suivre de modes de réalisation de celle-ci donnés en référence aux dessins dans lesquels :

- la figure 1 représente de façon schématique un dispositif selon l'invention en vue de dessus ;
- la figure 2 représente de manière schématique la coupe transversale A-A (agrandie) du dispositif de la figure 1 ;

- la figure 3 représente une vue de dessus en microscopie électronique d'un premier mode de réalisation d'une constriction d'un dispositif du type représenté aux figures 1 et 2;
- la figure 4 représente une vue en perspective en microscopie électronique de la constriction représentée à la figure 3 ;
- la figure 5 représente une vue de dessus en microscopie optique d'un second mode de réalisation d'une constriction d'un dispositif du type représenté aux figures 1 et 2 ;
- les figures 6A et 6B représentent schématiquement les champs de force hydrodynamique et électrophorétique dans le canal (hors et dans la constriction) ;
- les figures 7A, 7B et 7C représentent des graphes montrant les points d'arrêts de molécules en fonction de la vitesse électrophorétique de molécules et de la vitesse hydrodynamique appliqués ou du champ électrique et de la vitesse d'écoulement du fluide dans des fluides newtoniens et non-newtoniens;
- la figure 8 représente une photographie de microscopie de fluorescence de deux populations d'ADN différents par leur taille séparés et concentrés grâce à un dispositif du type de celui décrit en référence aux figure 1 à 4 ;
- la figure 9 représente une photographie de microscopie de fluorescence de trois types d'ADN séparés et concentrés grâce à un dispositif du type de celui décrit en référence aux figures 1 à 4 ;
- la figure 10 est un graphe montrant l'intensité lumineuse des points apparaissant dans le cadre figurant sur la figure 9 ;
- la figure 11 représente une photographie de microscopie de fluorescence de différents ADNs séparés et concentrés grâce à un dispositif du type de celui décrit en référence aux figures 1 à 4 ;
- la figure 12 est un graphe montrant l'intensité lumineuse des points apparaissant dans le cadre figurant sur la figure 11 ;
- la figure 13 représente une photographie de microscopie de fluorescence de billes en polystyrène de 100 nm de diamètre concentrées grâce à un dispositif du type de celui décrit en référence aux figures 1 à 4.

## Description de modes de réalisation du dispositif

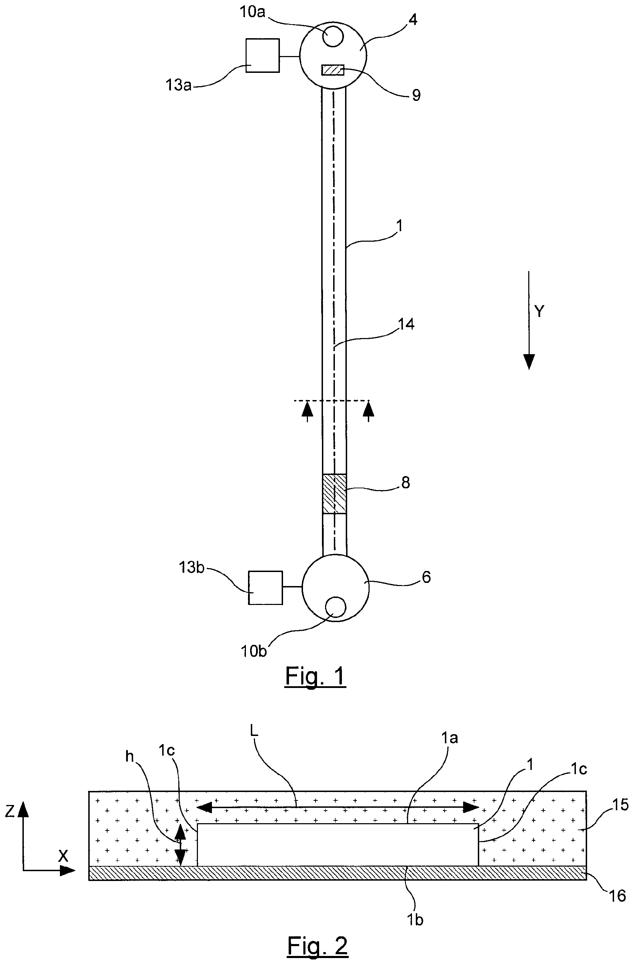

En faisant référence aux figures 1 à 5, le dispositif selon l'invention comporte un support 16, qui peut être une lame ou lamelle de verre, et une structure 15 présentant un évidement (par exemple une structure microfabriquée), qui peut être en silicium, scellée sur le support 16 de manière connue en soi. Par exemple, il est possible d'utiliser des techniques de dépôt de film, de photolithographie, de gravure (chimique ou plasma) et de collage. Le dépôt de film peut être effectué par centrifugation, par oxydation thermique, par dépôt chimique ou physique en phase vapeur (CVD et PVD), par CVD à basse pression, par CVD améliorée par plasma, par pulvérisation...

La structure 15 (avec son évidement) et le support 16 définissent un canal 1. D'une façon générale, c'est-à-dire sur la plupart de sa longueur, le canal 1 présente la forme d'un cylindre creux de section rectangulaire définie par une paroi supérieure 1a, une paroi inférieure 1b et des parois latérales 1c. L'axe principal du cylindre est l'axe d'écoulement 14 dans le canal 1. Perpendiculairement à cet axe d'écoulement 14, une première section transversale du canal 1 est définie par une hauteur notée h et une largeur notée L. La hauteur h correspond à la dimension minimale de la section transversale (c'est également la distance entre le support 16 et le fond de l'évidement de la structure 15), et la largeur L correspond à la dimension dans la direction orthogonale à celle de la hauteur.

En général, en utilisation, la hauteur h correspond à la verticale, tandis que la largeur L et l'axe d'écoulement 14 sont dans le plan horizontal. La valeur de h peut être choisie en fonction de la taille des molécules ou objets électriquement chargés à concentrer.

Le dispositif comprend par ailleurs une zone d'introduction 9 d'un échantillon dont on veut concentrer les molécules ou objets électriquement chargés et une zone de détection 8. Dans le cas présent, la zone d'introduction 9 de l'échantillon se situe dans le réservoir 4 et l'échantillon est injecté dans le réservoir grâce à un dispositif annexe.

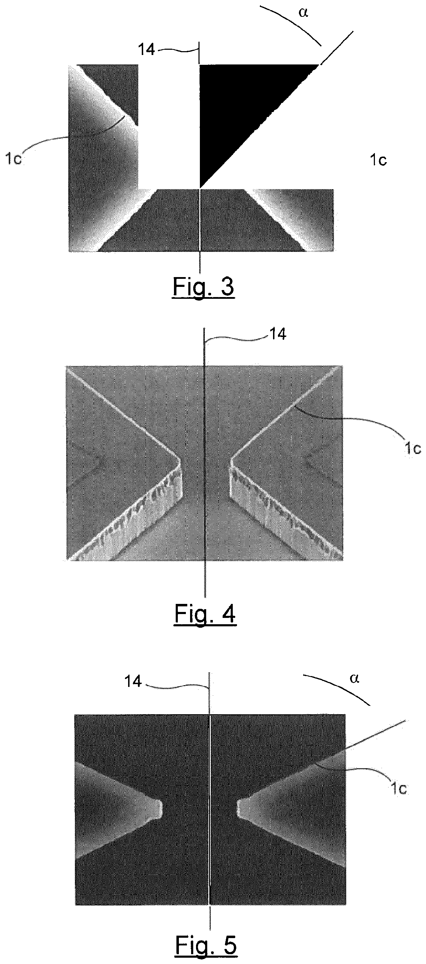

Selon l'invention, le canal 1 présente une constriction, représentée aux figures 3 et 4 ou 5, au niveau de la zone de détection 8 définissant une deuxième section transversale orthogonale à l'axe d'écoulement 14 dont la dimension minimale est inférieure d'au moins 10% ou de 20% ou de 50% ou de 95% de préférence à la dimension correspondante de ladite première section. Selon l'invention, la constriction a pour effet de moduler spatialement les amplitudes des champs électriques et hydrodynamiques le long de l'axe d'écoulement.

En référence aux figures 3 à 5, des vues par microscopie électronique de deux variantes de ladite constriction 2 sont représentées. Selon la variante de la figure 3 (vue de dessus) et 4 (vue en perspective) les parois latérales 1c du canal 1 forment au niveau de la constriction un angle $\alpha$ de 45° avec l'axe 14 du canal. Selon la variante de la figure 5, les parois latérales du canal 1 forment au niveau de la constriction un angle de 65 °. Au niveau de la constriction, la largeur du canal est réduite à 2,5 $\mu$m. Ainsi, selon l'invention, le dispositif est alors une travée de hauteur constante creusée dans le silicium et dont la largeur est modulée.

En utilisation, le canal 1 est rempli d'une solution, qui est adaptée à l'électrophorèse dans laquelle on a ajouté des éléments permettant de rendre le fluide non-newtonien. Un échantillon contenant des molécules ou objets électriquement chargés à concentrer est introduit dans le dispositif dans la zone d'introduction 9.

On met en oeuvre la migration des molécules ou objets électriquement chargés dans le canal 1, le long de l'axe d'écoulement 14, du premier réservoir 4 vers le deuxième réservoir 6. Pour ce faire, un flux hydrodynamique est généré dans le canal 1 (notamment par les moyens de contrôle de pression 13a, 13b du premier réservoir 4 et du deuxième réservoir 6).

Conjointement, un champ électrique est généré dans le canal 1 au moyen des électrodes 10a, 10b dans les réservoirs 4, 6 respectivement. Ce champ électrique est adapté pour appliquer une force électrostatique sur les molécules ou objets électriquement chargés à concentrer qui tend à les déplacer dans la direction opposée au flux hydrodynamique appliqué.

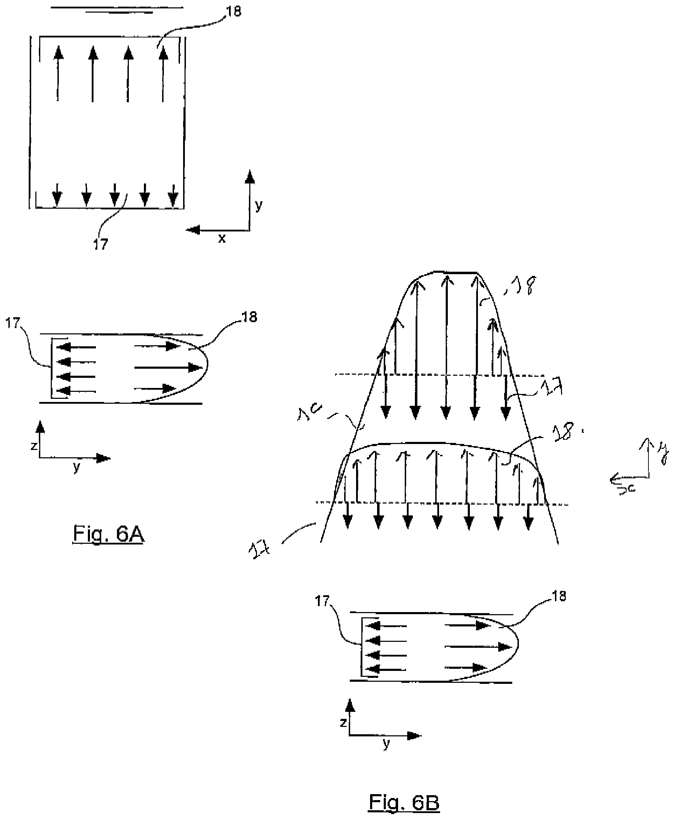

En référence à la figure 6A (la partie supérieure de la figure étant une vue de dessus, et la partie inférieure une vue de côté), lors de la mise en oeuvre du dispositif en dehors de la constriction présente dans la zone de détection 8, le champ de force électrophorétique 17 dans le canal 1 est essentiellement uniforme, aussi bien dans la direction de l'axe d'écoulement 14 (axe y), que selon la hauteur h (axe z) et la largeur L (axe x) du canal 1, sauf au voisinage immédiat des parois du canal (sur une longueur caractéristique négligeable par rapport aux dimensions h et L). En ce qui concerne le champ de force hydrodynamique 18, celui-ci est uniforme dans la direction de l'axe d'écoulement 14 (axe y) et selon la largeur L (axe x) sauf au voisinage immédiat des parois du canal (sur une longueur caractéristique négligeable par rapport à la dimension L). En revanche, il n'est pas uniforme selon la hauteur h (axe z). Généralement, il présente un profil de type parabolique caractéristique de la loi de Poiseuille. Le rapport d'aspect L/h du canal 1 est choisi afin d'obtenir cette configuration du champ de force hydrodynamique : c'est pourquoi le rapport d'aspect est généralement supérieur ou égal à 1, ou à 10 ou à 20. La non-uniformité du champ de force hydrodynamique 18 selon la hauteur h a une influence sur l'efficacité de la concentration des molécules ou objets électriquement chargés.

Les profils souhaités de flux hydrodynamique (caractérisés notamment par des valeurs de débit et de vitesse moyenne données), sont obtenus en actionnant les moyens respectifs de contrôle de pression 13a, 13b, de sorte à générer une différence de pression entre le réservoir d'entrée et le réservoir de sortie. Par exemple, pour générer le flux hydrodynamique assurant la migration des molécules ou objets déformables électriquement chargés à concentrer de la zone d'introduction 9 vers la zone de détection 8, on génère une différence de pression entre le réservoir 4 et le réservoir 6. Par exemple, compte tenu de la géométrie des canaux, une différence de pression de 0,01 à 10 bar, de préférence de 0,05 à 4 bar, et de manière plus particulièrement préférée de 0,1 à 1 bar, permet d'obtenir les profils de flux hydrodynamique souhaités.

La figure 6B représente une vue de dessus et une vue de côté du canal dans la zone de détection comportant la constriction. La partie supérieure de la figure représente une vue de dessus du canal dans lequel la largeur du canal diminue le long de l'axe d'écoulement dû à la présence des parois latérales 1c. La partie inférieure représente une vue de côté du canal, la hauteur du canal restant inchangée par la présence de la constriction. En référence à la partie supérieure de la figure, dans le plan (x,y), on note que la valeur du champ électrophorétique augmente lorsque la largeur diminue. De même, le profil du champ hydrodynamique évolue par conservation du flux hydrodynamique le long de la zone de constriction. Ainsi, la variation de la largeur du canal induit une variation de la valeur uniforme du champ électrique et une variation du profil de champ hydrodynamique le long de l'axe de l'écoulement. En revanche, sur la partie inférieure de la figure, dans le plan (y,z), le champ hydrodynamique et le champ électrophorétique restent invariant le long de l'axe d'écoulement.

Chaque molécule est donc soumise, en fonction de sa position dans la zone de détection, à différents champs hydrodynamiques et électrophorétiques. La vitesse de propagation de chaque molécule ou objet chargé électriquement varie donc lors de son déplacement dans la zone de constriction le long de l'axe d'écoulement.

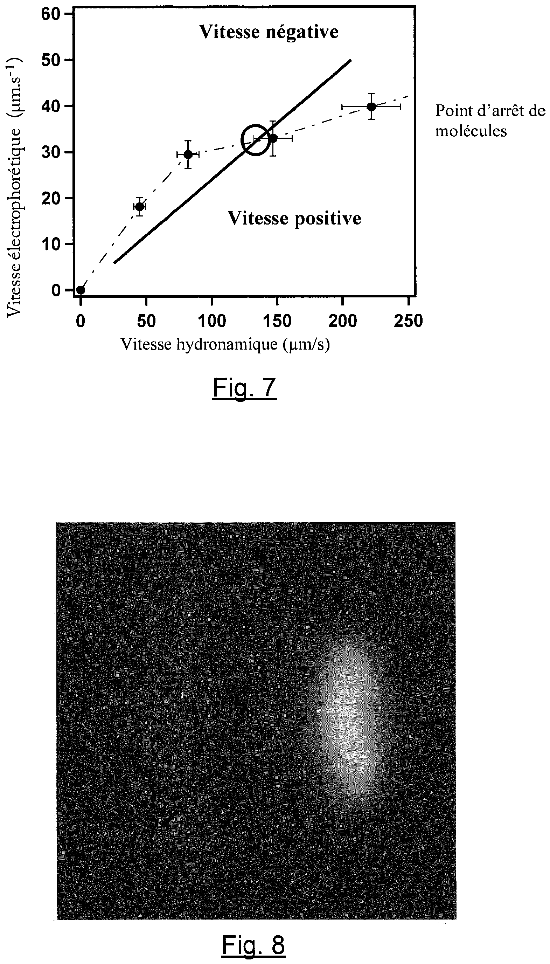

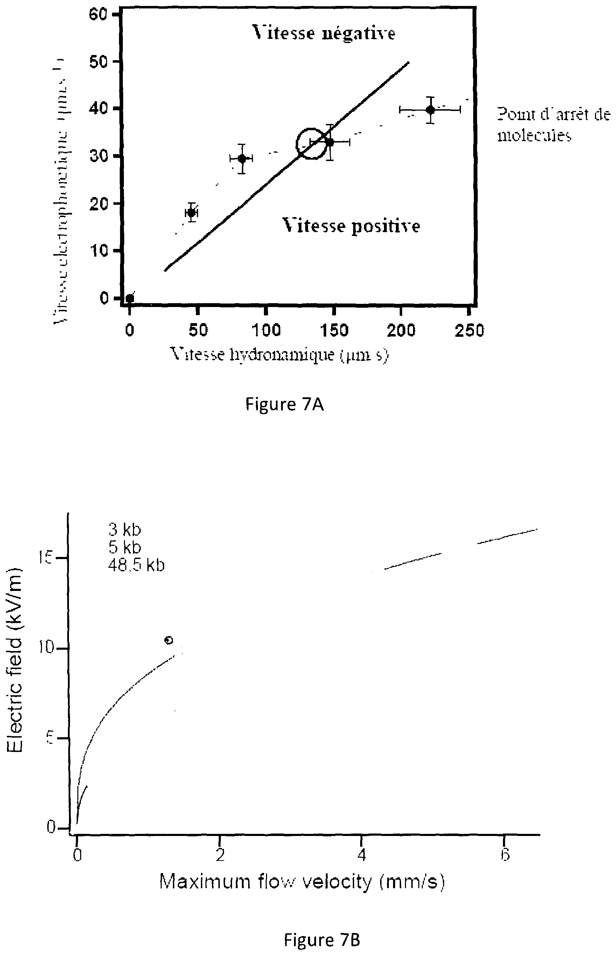

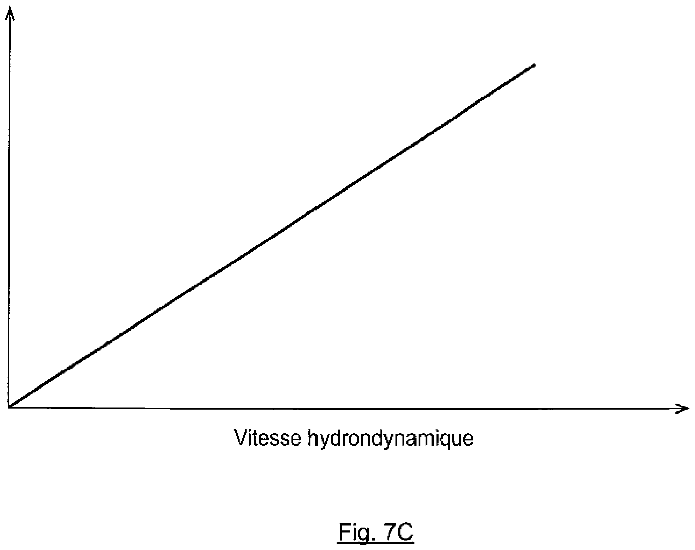

En référence aux figures 7 A, 7B et 7C, le principe à la base de l'invention est expliqué.

Un échantillon contenant des molécules d'ADN comportant 48.5 kpb a été introduit dans un dispositif du type de celui décrit ci-dessus mais ne présentant pas de constriction, le canal du dispositif en question présentant une section de dimension minimale de 2 μm. Cette introduction a été effectuée de façon à observer un flux de molécules net constant. Les plages de vitesses électrophorétiques (Ve) et hydrodynamiques (Vh) ont été balayées en faisant varier le champ électrophorétique ainsi que le différentiel de pression hydrostatique appliqués sur le dispositif. On a constaté de façon expérimentale que la molécule d'ADN était stoppée pour différents couples de valeurs de vitesse hydrodynamiques (vh) et électrophorétiques (ve) représentés sur la courbe de la figure 7A en pointillés. Au-dessous de cette courbe, il existe un effet hydrodynamique dominant, correspondant à une vitesse positive des molécules tandis qu'au-dessus de cette courbe, la vitesse électrophorétique domine, correspondant à une vitesse globale négative et les molécules reculent. L'utilisation selon l'invention d'une constriction du canal est symbolisée sur le graphe par la droite portée passant par l'origine qui permet de balayer différentes valeurs de vitesse hydrodynamique et électrophorétique comme expliqué en référence à la figure 6B et aux figures 7B et 7C.

L'endroit où cette droite coupe la courbe regroupant les points pour laquelle la vitesse est nulle (représenté par un cercle sur la figure 7A) correspond à une zone d'arrêt unique pour un type de molécule ou objets électriquement chargé donné.

On constate, par ailleurs, en référence à la figure 7B, que, pour différents échantillons d'ADN de taille différente soit 3kb, 5kb et 48,5 kb, les courbes illustrant les paramètres d'arrêt des molécules en fonction de la vitesse du fluide et du champ électrique appliqué sont différentes et dépendent de la taille de la molécule d'ADN considérée.

Ainsi, en balayant différentes valeurs de vitesse électrophorétique et hydrodynamique au sein de la constriction, comme représenté par la flèche, il est possible de concentrer et de stopper différentes molécules d'ADN à des endroits différents.

Sans vouloir être liés par une théorie, les inventeurs estiment qu'en présence d'un fluide non-newtonien, le cisaillement du fluide engendre des couplages non linéaires entre l'écoulement hydrodynamique et la mobilité électrophorétique via une force transverse qui tend à plaquer les molécules vers la paroi. Cette force transverse explique la croissance molle de la courbe de réponse de l'ADN : plus la vitesse de l'écoulement est rapide, plus le champ électrique nécessaire pour arrêter une molécule est d'intensité faible. Cette force est, de plus, dépendante de la taille de l'ADN manipulé, puisqu'une longue molécule induit une perturbation plus importante de l'écoulement et donc un effet non newtonien accru. Aussi, pour des paramètres d'actionnement des fluides donnés (champ électrique et différentiel de pression hydrostatique), il existe une série de points d'arrêt des molécules permettant de les stopper à une position différente le long de la canalisation.

Le dispositif selon l'invention, en présence d'un fluide non-newtonien, permet donc de concentrer et de séparer des objets électriquement chargés de taille différente, même ceux de mobilité semblable (à l'instar des dispositifs de l'art antérieur).

En présence d'un fluide newtonien, comme nous allons le démontrer, il n'est pas possible de concentrer des objets électriquement chargés ni de les séparer.

En effet, dans un fluide newtonien, la vitesse électrophorétique dépend linéairement du champ électrique selon la relation ve(y)=$\mu$E(y), où $\mu$ est la mobilité électrophorétique de l'ADN qui ne dépend pas de la taille de la chaîne, et la vitesse hydrodynamique est égale à la vitesse moyenne du fluide selon la position y de la molécule , soit vh(y)= v(y), y étant la direction d'écoulement du fluide dans le canal. Les molécules seront donc stoppées lorsque ve(y)=vh(y) soit E(y)=1/$\mu$ v(y). La courbe d'arrêt, dans le cas d'un fluide non-newtonien, est donc une droite passant par l'origine et de pente 1/$\mu$ comme illustré dans la figure 7C.

On considère maintenant les variations de champ électrique et de vitesse hydrodynamique pouvant être induits par une constriction. Le champ électrique E et la vitesse d'écoulement v étant à flux conservatif, en première approximation, on peut considérer que :

$$v(y)=S0/S(y).v0$$

$$E(y)=S0/S(y).E0$$

où S(y) est la section du canal dans la constriction à la position y ; et S0, E0, v0 désignent les valeurs de la section du canal, du champ électrique et de la vitesse du fluide loin de la constriction, en l'absence de la perturbation induite par la contriction.

La constriction permet donc de balayer un ensemble de couples champ électrique/vitesse d'écoulement sur une droite passant par l'origine dont la pente vaut E0/v0, E0 étant le champ électrique appliqué dans le canal et v0 la vitesse d'écoulement dans le canal en l'absence de constriction.

Ainsi, pour pouvoir stopper une molécule, il faut appliquer, en l'absence ou en présence de constriction, des conditions expérimentales telles que E0/v0=1/$\mu$. En revanche, cette molécule ne pourrait se déplacer dans le fluide pour pouvoir être concentrée puisque sa vitesse serait invariante et nulle dans tout le canal. En effet, pour pouvoir concentrer des molécules au même endroit, il est nécessaire que la vitesse de déplacement globale de la molécule ve+vh diminue avant de s'annuler au point d'arrêt. On verra par la suite, que lorsque l'échantillon comporte plusieurs molécules ou objets électriquement chargés, par exemple de mobilité semblable mais de taille différente telles que des molécules d'ADN, lorsque les paramètres tels que l'angle de constriction et les valeurs des champs hydrodynamiques et électrophorétiques appliqués sont choisis de façon appropriée, il est possible de stopper les différentes molécules ou objets à différentes positions le long de l'axe d'écoulement lorsque ceux-ci sont dissous dans un fluide non-newtonien. Dans ce cas, l'invention permet également de séparer les molécules ou objets électriquement chargés de mobilité semblable. Les moyens de détection ne sont pas représentés sur les figures. Ils peuvent comprendre un objectif de microscope du côté du support 16 opposé au canal 1, et un détecteur relié à celui-ci, tel qu'une caméra CCD. Les molécules individuelles ou les objets individuels électriquement chargés peuvent ainsi être détectés sur l'image acquise, et une mesure d'intensité globale dans la zone de détection 8 ou une partie de celle-ci en fonction du temps peut être effectuée.

Des moyens d'analyse des mesures et de présentation des données obtenues peuvent être associés à ce dispositif.

Le dispositif peut aussi être intégré dans un laboratoire sur puce, comprenant par exemple d'autres canaux, réservoirs et / ou électrodes similaires à ceux décrits ci-dessus. Par exemple, le laboratoire sur puce peut comprendre un dispositif de réaction chimique ou biochimique, couplé, en aval, au dispositif de séparation selon l'invention. Ainsi, la mise en oeuvre du procédé de concentration selon l'invention permet d'analyser les produits d'une réaction chimique ou biochimique mise en oeuvre dans le laboratoire sur puce.

Le laboratoire sur puce peut également comprendre des moyens de collecte de fractions correspondant aux différentes molécules ou objets électriquement chargés concentrés. Ces moyens de collecte peuvent être prévus en aval de la zone de détection 8. Alternativement, ils peuvent remplacer la zone de détection 8, auquel cas le dispositif est utilisé dans un but uniquement préparatif.

Ces moyens de collecte peuvent être prévus en combinaison avec le dispositif de réaction chimique ou biochimique mentionné ci-dessus, ou sans celui-ci.

## Exemples

Une solution tampon standard pour l'électrophorèse d'ADN, constituée de 80 mM de trisHCL, 80 mM d'acide borique, 5 mM d'acide éthylène diamine tétraacétique, 0,5 $\mu$M de dithiothréitol, à laquelle on a ajouté 2 % en masse de PVP à 360 kDa, a été utilisée pour diluer différents échantillons d'ADN. Comme décrit précédemment, l'introduction du PVP permet de rendre la solution tampon non newtonienne. D'une manière générale, différents échantillons d'ADN de tailles différentes ont été dilués dans cette solution tampon à raison de 1 à 10 ng/$\mu$l en acides nucléiques.

Les acides nucléiques présents dans les fragments d'ADN sont rendus fluorescents par marquage à l'aide d'un agent intercalant (YOYO(R), Molecular Probes) à raison d'une sonde pour 4 paires de bases.

Le dispositif utilisé pour séparer et analyser cette population de molécules est tel que représenté sur la figure 1. Le canal 1 a une largeur de 100 $\mu$m et une hauteur de 2 $\mu$m. Dans sa zone la plus étroite formée par la constriction, aussi appelée seconde section transversale, le canal 1 a une largeur de 2 $\mu$m et une hauteur de 2 $\mu$m. La zone d'introduction 9 et la zone de détection 8 sont séparées de 5 mm. 200 $\mu$l de solution tampon ne contenant pas d'échantillon sont disposés dans les réservoirs 4, et 6 du canal 1. Un flux de solution du réservoir 4 vers le réservoir 6 est créé par ajustement des pressions afin de permettre la saturation des surfaces en PVP, et ce durant 30 min.

## Exemple 1 : échantillon contenant deux types d'ADN de petites et de grandes tailles

Dans cet exemple, l'invention est mise en oeuvre pour séparer et concentrer de l'ADN Lambda à 48,5 kpb et de l'ADN PhiX174 à 5,4 kpb contenu dans une solution tampon telle que décrite précédemment.

Une différence de pression globale de 100 mbar et une différence de tension globale de 40 V ont été mises en oeuvre avec des flux hydrodynamique et électrophorétique croisés, c'est-à-dire orientés selon l'axe d'écoulement mais selon des axes opposés. Après une minute d'attente, une photographie de microscopie de fluorescence a été prise. Cette photographie est représentée à la figure 8. Grâce à la constriction, les molécules ont été arrêtées dans une zone en amont de la constriction. Celles-ci s'accumulent car les effets hydrodynamiques dominent avant le point d'arrêt, ce qui conduit à une vitesse positive, et elles reculent en aval. Le dispositif constitue alors à la fois un concentrateur, et un séparateur car le point d'arrêt dépend de la taille des molécules. Sur la figure 8, on voit ainsi un nuage correspondant aux molécules d'ADN Lambda séparé d'un autre nuage correspondant aux molécules d'ADN PhiX174. Après une minute de mise en oeuvre du dispositif, un facteur 10 de concentration de ces deux différents ADN a été obtenu. Ce facteur peut être encore amélioré en augmentant le flux hydrodynamique.

## Exemple 2 : Echantillon contenant trois types d'ADN

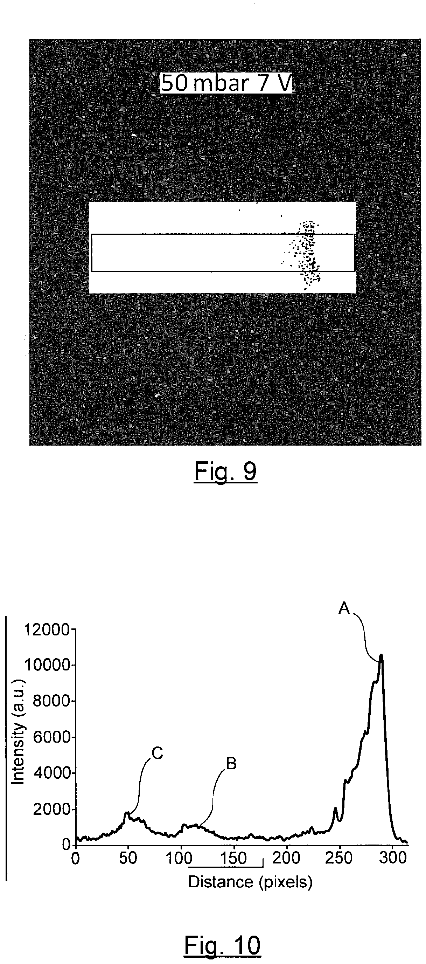

L'invention a aussi été mise en oeuvre avec un échantillon contenant trois types d'ADN, de 15 kbp, 35 kbp et 49bp. Une différence de pression globale de 50 mbar et une différence de tension globale de 7 V ont été utilisées avec des flux hydrodynamique et électrophorétique croisés. Après 100 secondes d'attente une photographie de microscopie de fluorescence a été prise. Cette photographie est représentée à la figure 9. L'intensité lumineuse des trois nuages correspondant à la concentration des trois types de molécules d'ADN a été évaluée. Les résultats correspondants sont portés sur le graphe représenté à la figure 10. Ce graphe montre trois pics A, B et C correspondant aux trois types d'ADN séparés et concentrés.

## Exemple 3 : échantillon contenant des ADN de petites tailles

L'invention a également été mise en oeuvre sur un marqueur de poids moléculaire ADN Ladder 1KpB comportant de 500 à 10 000 paires de bases.

Une différence de pression globale de 100 mbar et une différence de tension globale de 40 V ont été utilisées avec des flux hydrodynamique et électrophorétique croisés.

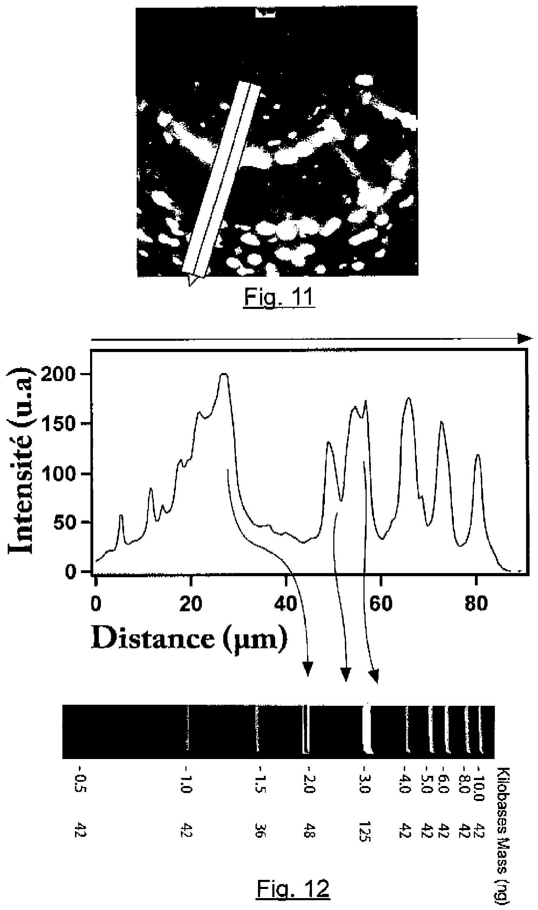

Après 17 secondes d'attente une photographie de microscopie de fluorescence a été prise. Cette photographie est représentée à la figure 11. L'intensité lumineuse de la zone incluse dans le cadre porté sur cette figure a été mesurée. Les résultats correspondants sont portés sur le graphe représenté à la figure 12. Ces résultats montrent une séparation et une concentration correspondant aux différents ADN constituant le marqueur, tels que ceux représentés sur l'échelle inférieure et implique que l'invention peut être mise en oeuvre pour l'application d'ADNs de courte taille..

## Exemple 4 : échantillon contenant des microbilles

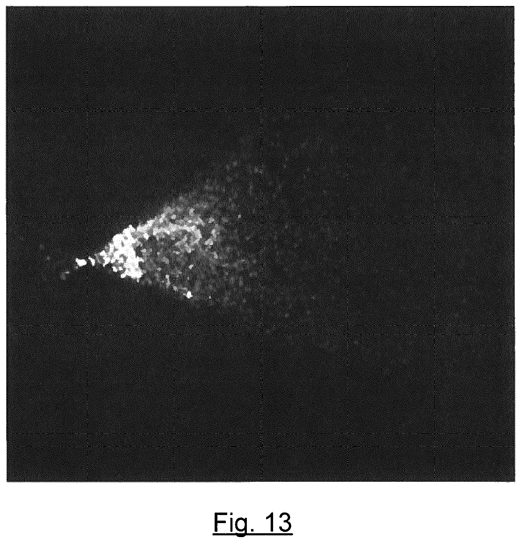

L'invention a également été mise en oeuvre avec un échantillon contenant des microbilles fluorescentes en polystyrène de 100 nm de diamètre (Fluorospheres™, société Invitrogen). La déformabilité de telles microbilles est assez faible, son module de Young étant de l'ordre de 3 GPa. Les microbilles ont été diluées dans une solution comportant la solution tampon précédemment citée et 2 % en masse de PVP 360 kDa. Le même dispositif que celui décrit précédemment pour la séparation et la concentration de l'ADN a été utilisé. Dans cet exemple, 200 μl de solution à base de TBE et de PVP telle que celle décrite précédemment a été utilisée. Une différence de pression globale de 150 mbar et une différence de tension globale de 40 V ont été utilisées avec des flux hydrodynamique et électrophorétique croisés. Après 18 secondes d'attente, une photographie de microscopie de fluorescence a été prise. Cette photographie est représentée à la figure 13. Les microbilles sont concentrées dans une zone moins homogène que dans le cas de molécules d'ADN. La dispersion observée dans la répartition des microsphères peut s'expliquer par l'inhomogénéité de l'échantillon observé par rapport aux populations d'ADN dont le nombre de paires de bases est fixe. Ainsi, il est démontré qu'une séparation et une concentration d'objets électriquement chargés avec un module de Young supérieur ou égal à $10^9$ Pa, est également possible. Ainsi, le domaine d'application de l'invention n'est pas limité à la concentration et à la séparation de molécules d'ADN mais peut s'étendre à tout type d'objet chargé électriquement du type de ceux décrits précédemment.

On notera que selon la composition de l'échantillon et les valeurs de champ électrophorétique et hydrodynamique, certaines molécules ou certains objets électriquement chargés peuvent conserver une vitesse positive de déplacement lors de leur déplacement le long de l'axe d'écoulement du canal. Ces molécules ne sont donc pas stoppées en amont de la constriction et peuvent être récoltées en aval. Le dispositif selon l'invention peut donc également être utilisé comme dispositif de filtrage de molécules.

Dans les différents modes de réalisation et exemples représentés ici, le canal présentait la forme d'un tube creux de section rectangulaire dont l'une des faces est transparente de façon à permettre la détection optique des molécules ou objets électriquement chargés.

On peut également considérer un canal constitué par la lumière d'un capillaire, c'est-à-dire d'une structure tubulaire, de section préférentiellement carrée ou circulaire réalisée dans un matériau transparent tel que par exemple le verre.

On notera par ailleurs que, dans d'autres modes de réalisation, la constriction pourra être constituée par des parois se rapprochant de l'axe d'écoulement de façon non linéaire.

## Revendications

1.  Procédé de concentration d'objets électriquement chargés en milieu liquide non-newtonien, comprenant :

l'introduction d'un échantillon contenant des objets électriquement chargés dans un canal (1) formant une constriction et présentant un axe d'écoulement, une première section transversale orthogonale à l'axe d'écoulement et au moins une deuxième section transversale orthogonale à l'axe d'écoulement formant ladite constriction, les objets électriquement chargés étant destinés à migrer le long de l'axe d'écoulement depuis un premier réservoir (4) vers un deuxième réservoir (6);
l'application d'un flux hydrodynamique dans un sens du canal (1) par des moyens de contrôle de pression (13a, 13b) du premier réservoir (4) et du deuxième réservoir (6), conjointement à l'application d'un champ électrique adapté pour appliquer une force électrostatique sur les objets électriquement chargés qui tend à les déplacer dans la direction opposée au flux hydrodynamique, le champ électrique ayant une valeur uniforme en dehors de la constriction, dans ledit canal par deux électrodes (10a, 10b) dans le premier et dans le deuxième réservoirs (4, 6), permettant de déplacer les objets électriquement chargés dans le canal selon l'axe d'écoulement de la première section vers la deuxième section et de les stopper et les concentrer dans au moins une zone en amont de ladite deuxième section transversale.

2.  Procédé selon la revendication 1 dans lequel le milieu liquide comprend des polymères non chargés.

3.  Procédé selon la revendication 2 dans lequel les polymères non chargés sont choisis parmi la polyvinylpyrrolidone (PVP), le poly-(éthylène glycol), le polyacrylamide et leurs mélanges.

4.  Procédé selon la revendication 3 dans lequel les polymères non chargés sont présents dans une concentration massique de 0,1 à 10 %, de préférence de 0,5 à 5 %, de manière plus particulièrement préférée de 1 à 4%.

5.  Procédé selon l'une des revendications précédentes dans lequel :

le champ électrique appliqué vaut de 10 V/m à 10000 V/m, de préférence de 100 V/m à 5000 V/m, et de manière plus particulièrement préférée de 200 V/m à 1000 V/m ; et / ou
le flux hydrodynamique est **caractérisé par** une vitesse moyenne de 1 à 10 000 μm/s, de préférence de 5 à 5 000 μm/s et de manière plus particulièrement préférée de 10 à 1 000 μm /s.

6.  Procédé selon l'une des revendications précédentes, dans lequel :

l'introduction des objets électriquement chargés est effectuée dans une zone d'introduction du canal et le déplacement des objets électriquement chargés est effectué de la zone d'introduction vers une zone de détection du canal, le procédé comprenant en outre :

la détection des objets électriquement chargés arrivant dans la zone de détection.

7. Procédé selon l'une quelconque des revendications précédentes **caractérisé en ce que** les dits objets électriquement chargés sont choisis dans le groupe constitué par : les molécules d'ADN simple ou double brin ou d'ARN comprenant au moins 20 bases ou paires de bases ; les peptides, polypeptides ou protéines comprenant au moins 100 motifs d'acides aminés; les glucides polymériques ou autres polymères ; les cellules caryotes ou procaryotes; les nanoobjets ou nanoparticules

8. Dispositif de concentration d'objets électriquement chargés en milieu liquide, comprenant:

un canal (1) formant une constriction et présentant un axe d'écoulement (14), les objets électriquement chargés étant destinés à migrer le long de l'axe d'écoulement depuis un premier réservoir (4) vers un deuxième réservoir (6);
le canal (1) étant rempli d'un milieu liquide non newtonien ;
des moyens d'application d'un flux hydrodynamique (13a, 13b) dans un sens dudit canal (1) par des moyens de contrôle de pression (13a, 13b) du premier réservoir (4) et du deuxième réservoir (6) ; et,
des moyens d'application dans le sens inverse d'un champ électrique (10a, 10b) de valeur uniforme, en dehors de la constriction, dans le canal (1), où ledit canal (14) présente une première section transversale orthogonale à l'axe d'écoulement et au moins une deuxième section transversale orthogonale à l'axe d'écoulement formant ladite constriction, et où
les moyens d'application du champ électrique (10a, 10b) sont dans le premier et dans le deuxième réservoirs (4, 6), permettant de déplacer les objets électriquement chargés dans le canal selon l'axe d'écoulement de la première section vers la deuxième section et de les stopper et les concentrer dans au moins une zone en amont de ladite deuxième section transversale.

9. Dispositif selon la revendication 8 où ledit canal a la forme d'un tube creux de section rectangulaire présentant une paroi inférieure, une paroi supérieure et deux parois latérales, lesdites parois latérales formant localement au moins une constriction.

10. Dispositif selon l'une des revendications 8 à 9 où ladite deuxième section présente une dimension minimale inférieure d'au moins 10% ou de 20% ou de 50% ou de 95% à la dimension correspondante de ladite première section.

11. Dispositif selon la revendication 9 ou 10 où lesdites parois latérales dudit tube creux présentent chacune une portion formant un angle compris entre 10° et 65 ° avec ledit axe d'écoulement (14).

12. Dispositif selon la revendication 8 où ledit canal est la lumière d'un capillaire présentant au moins une constriction.

13. Dispositif selon la revendication 12 où ledit capillaire présente une section carrée ou circulaire.

14. Dispositif selon la revendication 12 ou 13 où ledit capillaire présente une portion formant un angle compris entre 10° et 65 ° avec ledit axe d'écoulement (14).

**Patentansprüche**

1. Verfahren zum Konzentrieren elektrisch geladener Objekte in einem nicht-newtonschen flüssigen Medium, das Folgendes umfasst:

Einleiten einer Probe, die elektrisch geladene Objekte enthält, in einen Kanal (1), der eine Verengung bildet und eine Strömungsachse, einen ersten queren Abschnitt orthogonal zur Strömungsachse und mindestens einen zweiten queren Abschnitt orthogonal zu der Strömungsachse aufweist, der die Verengung bildet, wobei die elektrisch geladenen Objekte zum Wandern entlang der Strömungsachse von einem ersten Reservoir (4) zu einem zweiten Reservoir (6) bestimmt sind;
Applizieren eines hydrodynamischen Flusses in einer Richtung des Kanals (1) durch Drucksteuermittel (13a, 13b) des ersten Reservoirs (4) und des zweiten Reservoirs (6), zusammen mit dem Anlegen eines elektrischen Feldes, angepasst zum Ausüben einer elektrostatischen Kraft auf die elektrisch geladenen Objekte, die dazu neigt, sie in die dem hydrodynamischen Fluss entgegengesetzte Richtung in dem Kanal durch zwei Elektroden (10a, 10b) im ersten und im zweiten Reservoir (4, 6) zu bewegen, wobei das elektrische Feld außerhalb der Verengung einen gleichmäßigen Wert hat, wodurch die elektrisch geladenen Objekte in dem Kanal entlang der Strömungsachse vom ersten Abschnitt zum zweiten Abschnitt bewegt werden können und in mindestens einer Zone stromaufwärts des zweiten queren Abschnitts gestoppt und konzentriert werden können.

2. Verfahren nach Anspruch 1, wobei das flüssige Medium ungeladene Polymere umfasst.

3. Verfahren nach Anspruch 2, wobei die ungeladenen Polymere aus Polyvinylpyrrolidon (PVP), Polyethylenglycol, Polyacrylamid und deren Mischungen ausgewählt sind.

4. Verfahren nach Anspruch 3, wobei die ungeladenen Polymere in einer Massenkonzentration von 0,1 bis 10 %, vorzugsweise 0,5 bis 5 %, besonders bevorzugt 1 bis 4 %, vorliegen.

5. Verfahren nach einem der vorhergehenden Ansprüche, wobei:

das angelegte elektrische Feld 10 V/m bis 10 000 V/m, vorzugsweise 100 V/m bis 5 000 V/m und besonders bevorzugt 200 V/m bis 1 000 V/m beträgt; und/oder
der hydrodynamische Fluss durch eine mittlere Geschwindigkeit von 1 bis 10 000 $\mu$m/s, vorzugsweise 5 bis 5 000 $\mu$m/s und besonders bevorzugt 10 bis 1 000 $\mu$m/s gekennzeichnet ist.

6. Verfahren nach einem der vorhergehenden Ansprüche, wobei:
das Einleiten der elektrisch geladenen Objekte in eine Einleitungszone des Kanals erfolgt und die Bewegung der elektrisch geladenen Objekte von der Einleitungszone zu einer Erkennungszone des Kanals erfolgt, wobei das Verfahren ferner Folgendes umfasst:
Erkennen der in der Erkennungszone ankommenden elektrisch geladenen Objekte.

7. Verfahren nach einem der vorhergehenden Ansprüche, **dadurch gekennzeichnet, dass** die elektrisch geladenen Objekte ausgewählt sind aus der Gruppe bestehend aus Folgendem: einzel- oder doppelsträngigen DNA- oder RNA-Molekülen, die mindestens 20 Basen oder Basenpaare umfassen; Peptiden, Polypeptiden oder Proteinen, die mindestens 100 Aminosäureeinheiten umfassen; polymeren Kohlenhydraten oder anderen Polymeren; karyotischen oder prokaryotischen Zellen; Nanoobjekten oder Nanopartikeln.

8. Vorrichtung zur Konzentration von elektrisch geladenen Objekten in einem flüssigen Medium, die Folgendes umfasst:

einen Kanal (1), der eine Verengung bildet und eine Strömungsachse (14) aufweist, wobei die elektrisch geladenen Objekte zum Wandern entlang der Strömungsachse von einem ersten Reservoir (4) zu einem zweiten Reservoir (6) bestimmt sind;
wobei der Kanal (1) mit einem nicht-newtonschen flüssigen Medium gefüllt ist;
Mittel zum Applizieren eines hydrodynamischen Flusses (13a, 13b) in einer Richtung des Kanals (1) durch Drucksteuermittel (13a, 13b) des ersten Reservoirs (4) und des zweiten Reservoirs (6); und
Mittel zum Anlegen eines elektrischen Feldes (10a, 10b) mit gleichmäßigem Wert in der Gegenrichtung, außerhalb der Verengung, in dem Kanal (1), wo der Kanal (14) einen ersten queren Abschnitt orthogonal zur Strömungsachse und mindestens einen zweiten queren Abschnitt orthogonal zur Strömungsachse aufweist, der die Verengung bildet,
und wo die Mittel zum Anlegen des elektrischen Feldes (10a, 10b) im ersten und im zweiten Reservoir (4, 6) sind, wodurch die elektrisch geladenen Objekte in dem Kanal entlang der Strömungsachse vom ersten Abschnitt zum zweiten Abschnitt bewegt werden können und in mindestens einer Zone stromaufwärts vom zweiten queren Abschnitt gestoppt und konzentriert werden können.

9. Vorrichtung nach Anspruch 8, worin der Kanal die Form eines hohlen Rohrs mit rechteckigem Querschnitt aufweist, das eine untere Wand, eine obere Wand und zwei Seitenwände aufweist, wobei die Seitenwände lokal mindestens eine Verengung bilden.

10. Vorrichtung nach einem der Ansprüche 8 bis 9, wobei der zweite Abschnitt eine Mindestabmessung aufweist, die um mindestens 10 % oder um 20 % oder um 50 % oder um 95 % kleiner ist als die entsprechende Abmessung des ersten Abschnitts.

11. Vorrichtung nach Anspruch 9 oder 10, worin die Seitenwände des hohlen Rohrs jeweils einen Teil aufweisen, der einen Winkel zwischen 10° und 65° mit der Strömungsachse (14) bildet.

12. Vorrichtung nach Anspruch 8, worin der Kanal das Lumen einer Kapillare mit mindestens einer Verengung ist.

13. Vorrichtung nach Anspruch 12, worin die Kapillare einen quadratischen oder kreisförmigen Querschnitt aufweist.

14. Vorrichtung nach Anspruch 12 oder 13, worin die Kapillare einen Teil aufweist, der einen Winkel zwischen 10° und 65° mit der Strömungsachse (14) bildet.

**Claims**

1. A method for concentrating electrically charged objects in a non-newtonian liquid medium, comprising:

   the introduction of a sample containing electrically charged objects into a channel (1) forming a constriction and having a flow axis, a first transverse section orthogonal to the flow axis and at least one second transverse section orthogonal to the flow axis forming said constriction, the electrically charged objects being intended to migrate along the flow axis, from a first reservoir (4) toward a second reservoir (6);
   the application of a hydrodynamic flow in a direction of the channel (1) by pressure control means (13a, 13b) of the first reservoir (4) and of the second reservoir (6), in conjunction with the application of an electric field adapted for applying an electrostatic force to the electrically charged objects, which tends to displace them in the direction opposed to the hydrodynamic flow, the electric field being of uniform value outside of the constriction, in said channel by two electrodes (10a, 10b) in the first and in the second reservoir (4, 6), making it possible to displace the electrically charged objects in the channel along the flow axis of the first section toward the second section and to halt them and to concentrate them in at least one zone upstream of said second transverse section.

2. The method according to claim 1, wherein the liquid medium comprises uncharged polymers.

3. The method according to claim 2, wherein the uncharged polymers are chosen from polyvinylpyrrolidone (PVP), polyethylene glycol), polyacrylamide and their mixtures.

4. The method according to claim 3, wherein the uncharged polymers are present in a concentration by weight of 0.1 to 10%, preferably of 0.5 to 5% and more particularly preferably of 1 to 4%.

5. The method according to one of the preceding claims, wherein:

   the applied electric field has a value of 10 V/m to 10,000 V/m, preferably of 100 V/m to 5,000 V/m and more particularly preferably of 200 V/m to 1,000 V/m; and/or
   the hydrodynamic flow is **characterised by** a mean velocity of 1 to 10,000 $\mu$m/s, preferably of 5 to 5,000 $\mu$m/s and more particularly preferably of 10 to 1,000 $\mu$m/s.

6. The method according to one of the preceding claims, wherein:
   the introduction of the electrically charged objects is carried out in an introduction zone of the channel and the displacement of the electrically charged objects is carried out from the introduction zone toward a detection zone of the channel, the method further comprising:
   the detection of the electrically charged objects arriving in the detection zone.

7. The method according to any one of the preceding claims, **characterised in that** said electrically charged objects are chosen from the group consisting of: single- or doublestranded DNA or RNA molecules comprising at least 20 bases or base pairs; peptides, polypeptides or proteins comprising at least 100 amino acid units; polymeric carbohydrates or other polymers; karyotic or prokaryotic cells; nanoobjects or nanoparticles.

8. A device for concentrating electrically charged objects in a liquid medium, comprising:

   a channel (1) forming a constriction and having a flow axis (14), the electrically charged objects being intended to migrate along the flow axis, from a first reservoir (4) toward a second reservoir (6);
   the channel (1) being filled with non-newtonian liquid medium;
   means for application of a hydrodynamic flow (13a, 13b) in a direction of said channel (1) by pressure control means (13a, 13b) of the first reservoir (4) and of the second reservoir (6); and
   means for application, in the reverse direction, outside of the constriction, of an electric field (10a, 10b) of uniform value in the channel (1), where said channel (14) has a first transverse section orthogonal to the flow axis and at least one second transverse section orthogonal to the flow axis, forming said constriction,
   and where the means for application of the electric field (10a, 10b) are in the first and in the second reservoir (4, 6), making it possible to displace the electrically charged objects in the channel along the flow axis of the first section toward the second section and to halt them and to concentrate them in at least one zone upstream of said second transverse section.

9. The device according to claim 8, where said channel has the shape of a hollow tube of rectangular cross-section having a lower wall, an upper wall and two side walls, said side walls locally forming at least one constriction.

10. The device according to one of claims 8 and 9, where said second section has a minimum dimension lower by at least 10 % or by 20 % or by 50 % or by 95 % than the corresponding dimension of said first section.

**11.** The device according to claim 9 or 10, where said side walls of said hollow tube each have a portion forming an angle of between 10° and 65° with said flow axis (14).

**12.** The device according to claim 8, where said channel is the lumen of a capillary having at least one constriction.

**13.** The device according to claim 12, where said capillary has a square or circular cross-section.

**14.** The device according to claim 12 or 13, where said capillary has a portion forming an angle of between 10° and 65° with said flow axis (14).

Fig. 1

Fig. 2

Fig. 3

Fig. 4

Fig. 5

Fig. 7

Fig. 8

**Fig. 9**

**Fig. 10**

Fig. 11

Fig. 12

17

Fig. 6A

Fig. 6B

Fig. 13

Figure 7A

Figure 7B

Vitesse hydrondynamique

Fig. 7C

**EP 3 194 952 B1**

## RÉFÉRENCES CITÉES DANS LA DESCRIPTION

**Documents brevets cités dans la description**

- WO 2014020271 A
- US 8753492 B
- US 8440063 B
- US 8783466 B
- US 2008087546 A1
- US 2005126914 A1